# EUROPEAN PATENT SPECIFICATION

(11) **EP 2 813 065 B1**
(45) Date of publication and mention of the grant of the patent: **30.08.2017**
(21) Application number: 13704866.6
(22) Date of filing: 05.02.2013
(51) Int. Cl.: A61B 1/00, H04N 5/225

(54) **CAMERA SYSTEM CONTROLLED BY A TABLET COMPUTER**
TABLET-COMPUTER GESTEUERTES KAMERASYSTEM
SYSTÈME À CAMÉRA CONTROLLÉ PAR UNE TABLETTE NUMÉRIQUE

(30) Priority: 07.02.2012 US 201261595827 P; 24.01.2013 US 201313749423
(43) Date of publication of application: 17.12.2014
(73) Proprietor: Arthrex, Inc, Naples, FL 34108 (US)
(72) Inventor: KENNEDY, Bruce, Santa Barbara, CA 93111 (US); DEPPMEIER, Tom, Santa Barbara, CA 93111 (US); SPEIER, Craig, Santa Barbara, CA 93111 (US)
(74) Representative: Lohr, Georg
(86) International application number: PCT/US2013/024771
(87) International publication number: WO 2013/119567

(56) References cited:
- JP-A- 2006 198 241
- US-A1- 2002 067 408
- US-A1- 2003 164 819
- US-A1- 2006 288 234
- US-B2- 7 520 853

## Description

### BACKGROUND OF THE INVENTION

This invention relates generally to medical camera systems and, more particularly, to endoscopic camera systems for use in a surgical environment.

One of the advances in recent years in surgical procedures is the availability of specialized imaging devices. These devices provide a surgeon with a view of the area of the body being worked on without the necessity of fully opening up the area, thereby allowing for less invasive surgical procedures. Generally, these devices consist of a camera head coupled to a camera control unit and an accompanying illuminator module.

Because of the requirements to be compatible with the operating room environment and the specific needs as to illumination and handling by the surgeon, these imaging devices are typically highly customized devices. Both the camera units and the electronic interface units are purpose-specific designs.

The camera control unit is typically coupled to at least one input device such as a mouse, keyboard, or touchscreen monitor. The camera control unit is typically also coupled to a surgical monitor for displaying images generated by the camera head. Typical camera controllers have either push button controls on the face plate, controls via buttons on the camera head and/or very limited graphic user interface (GUI) feedback on the surgical display. Thus, the input and display devices are limited in their functionality. Accordingly, there is a need for an improved medical camera system with improved input and display devices.

### SUMMARY OF THE INVENTION

The present invention is directed to a camera system that remedies the shortcomings of the prior art. A camera system according to an embodiment of the present invention has a camera head; a camera control unit coupleable to the camera head; a monitor coupleable to the camera control unit for displaying images from the camera head and information from the camera control unit; and a tablet computer coupleable to the camera control unit for control of the camera control unit and the camera head, the tablet computer further comprising an application that is authenticated by the camera control unit for pairing the tablet computer to the camera control unit.

Additionally, the tablet computer may have a memory for storing user information for use in controlling the camera control unit; a camera; and face recognition software for automatically identifying a tablet computer user and accessing the stored information for that user. Optionally, the memory for storing user information contains defined levels of authority and wherein once a user is identified using the face recognition software, the application loads the defined level of authority for that user and thereby limits the functionality of the application depending on the user.

In an embodiment, the tablet computer is initially coupled to the camera control unit by a physical connection for authentication and thereafter the tablet computer is coupleable to the camera control unit wirelessly. The camera control unit and the tablet computer may each comprise a database having at least one of the group consisting of user, surgeon, facility and system settings, the camera control unit database and the tablet computer database being synchronized when the camera control unit and the tablet computer are coupled together. Additionally, the system may have a database remotely located from the camera control unit and the tablet computer and wherein the camera control unit database and the tablet computer database are synchronized to the remotely located database.

Optionally, the tablet computer application locks out all other functionality on the tablet upon connection with the camera control unit. The tablet computer may also have a camera and the tablet computer application may allow for video conferencing using the tablet computer camera and the monitor.

Optionally, the tablet computer receives encrypted images from the camera control unit and the tablet computer application decrypts the images and creates a patient or surgeon report with at least a subset of the images for display or communication. Additionally, the tablet computer may have a global positioning system and pairing of the tablet computer to the camera control unit is dependent on the geographic location of the tablet computer.

Optionally, the tablet computer may be used to view live images from the camera head and when the tablet computer is displaying live images from the camera head an indication is displayed on the monitor. The tablet computer may transfer software or firmware updates to the camera control unit. The tablet computer may transfer software or firmware updates to the camera head. Optionally, the tablet computer may have a microphone and the application may allow a user to dictate notes.

Additionally, the present invention is directed to a method for authenticating a tablet computer for use with a camera system having a camera control unit, a monitor and a tablet computer, the method comprising the steps of: providing power to the camera control unit; starting an application on the tablet computer; establishing communication between the tablet computer and the camera control unit; determining the authenticity of the application on the tablet computer; requesting authentication identification from the camera control unit; displaying authentication identification on the monitor by the camera control unit; receiving the authentication identification on the tablet computer; and issuing an authentication certificate to the tablet computer, the authentication certificate allowing for pairing of the camera control unit and the tablet computer.

Communication between the camera control unit and the tablet computer may be wireless. The method may also include the steps of: prior to establishing communication between the tablet computer and the camera control unit, physically connecting the camera control unit and the tablet computer together; and after issuing an authentication certificate to the tablet computer, physically disconnecting the camera control unit and the tablet computer.

The present invention according to another embodiment is directed to a camera system having: a camera head; a camera control unit coupleable to the camera head; a monitor coupleable to the camera control unit for displaying images from the camera head and information from the camera control unit; a tablet computer coupleable to the camera control unit for control of the camera control unit and the camera head, the tablet computer further comprising an application that is authenticated by the camera control unit for pairing the tablet computer to the camera control unit; and at least one additional device coupleable to the camera control unit. The tablet computer is usable to control the camera control unit and the at least one additional device. The tablet computer may transfer software or firmware updates to the camera control unit. The tablet computer may transfer software or firmware updates to the at least one additional device.

### BRIEF DESCRIPTION OF THE DRAWINGS

The features, aspects and advantages of the present invention will become better understood with regard to the following description, appended claims and accompanying figures wherein:
FIG. 1 is a schematic diagram of a camera system according to an embodiment of the present invention;
FIG. 2 is a schematic diagram of a camera control unit and camera head usable in the camera system of FIG. 1;
FIG. 3 is a schematic diagram of a tablet computer usable in the camera system of FIG. 1; and
FIG. 4 is a flowchart showing a tablet computer authentication process in the camera system of FIG. 1 according to an embodiment of the present invention.

### DETAILED DESCRIPTION OF THE PREFERRED EMBODIMENTS

In the following description of the preferred embodiments, reference is made to the accompanying drawings which show by way of illustration specific embodiments in which the invention may be practiced. Wherever possible, the same reference numbers will be used throughout the drawings to refer to the same or like parts. It is to be understood that other embodiments may be utilized and structural and functional changes may be made without departing from the scope of the present invention.

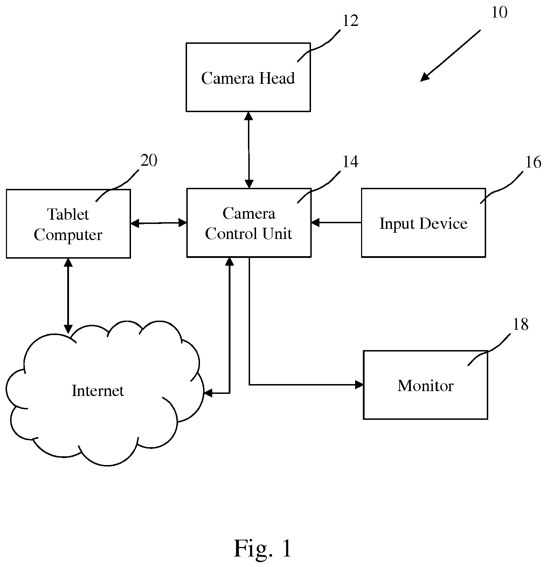

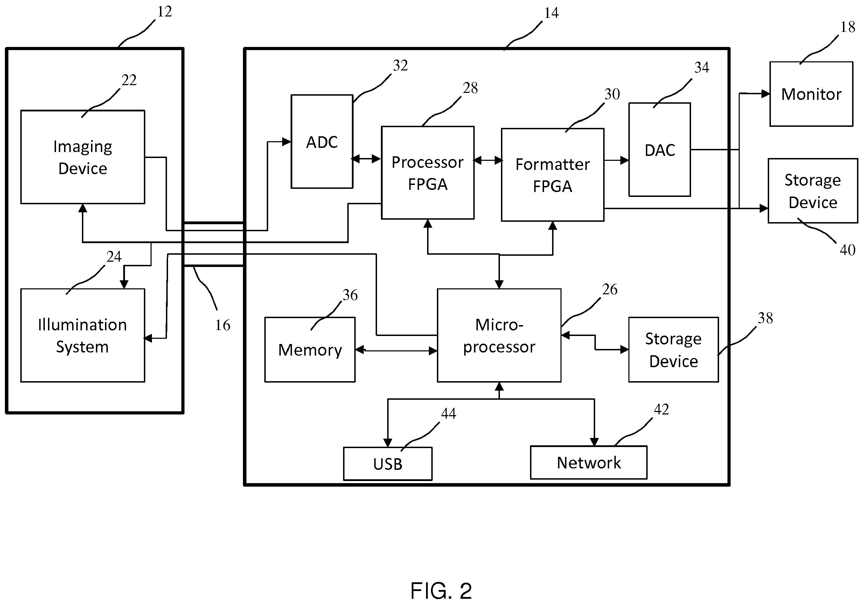

With reference to Figs. 1 and 2, an endoscopic camera system 10 according to an embodiment of the present invention has at least one camera head 12 connectable to a camera control unit 14 ("CCU"). The camera head 12 and camera control unit 14 may be connected via wire or wirelessly. The camera control unit 14 is also connectable to at least one input device 16 such as a mouse, keyboard, touchpad, or touchscreen monitor. Additionally, the camera control unit is connectable to a monitor 18. Additionally, the camera control unit is connectable to at least one tablet computer 20.

As shown in Fig. 2, the camera head 12 has an imaging device 22 which may include NTSC/PAL, single chip, three chip, standard definition, high definition, CCD and CMOS devices. The camera head may also have an illumination system 24. The camera head 12 may also have memory for storing camera data, camera control unit processing data or other information. The camera head 12 may also have a user input means such as buttons to control aspects of image capture.

As shown in Fig. 2, the camera control unit 14 contains a microprocessor 26 for interfacing with user input devices 16, a signal processing circuit 28, a signal formatting circuit 30, analog to digital converters 32, digital to analog converters 34 and memory 36. The camera control unit 14 runs program applications providing for a variety of capabilities. For example, the camera control unit 14 may provide for a live feed of the image generated by the camera head 12 to be displayed through the monitor 18. Additionally, the camera control unit 14 may provide image capture functionality allowing for images generated by the camera head 12 to be saved to a storage device, such as an internal storage device 38 or a storage device 40 external to the camera control unit. The captured images may be annotated and/or edited and displayed through the monitor 18.

In an embodiment, the camera control unit 14 also has at least one network interface 42 which may be a wired interface such as Ethernet, or a wireless network connection that allows for the camera control unit to access a network. Optionally, the network interface 42 allows the camera control unit 14 to access the Internet. The camera control unit 14 may be programmed to act as a web host, thereby allowing remote control of the camera control unit, such as via a web browser. This may allow the camera control unit to be accessed anywhere within a local network, or if allowed, from anywhere on the Internet, and both programmed and monitored. This may also allow for streaming video of a procedure to be observed in real-time anywhere in the world. In an embodiment, when streaming is taking place, an indication is made on the monitor 18.

In an embodiment, the external storage device 40 may be, for example, a flash memory storage device or a hard disk storage device, and may be connected to the camera control unit 14 through a USB connection 44 or firewire connection (not shown). In an embodiment, a program application for the camera control unit, or data relevant to a particular patient or surgeon, is stored on the external storage device 40 and may be used to quickly configure the camera control unit for future sessions. Preferably, the camera control unit 14 can save images and video in different formats and to different places (e.g. internal memory, an external memory, or to a remote location over the Internet).

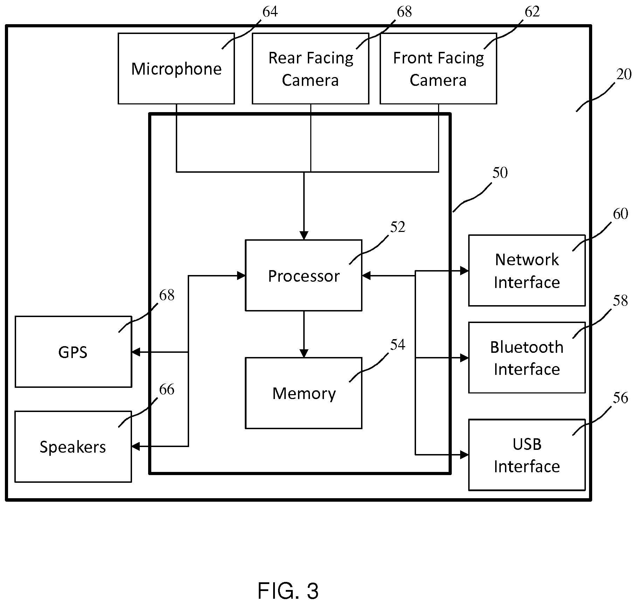

With reference to Fig. 3, as used herein, the terms "tablet" and "tablet computer" are used interchangeably and refer to a mobile computer having an integrated flat touch screen 50, a processor 52 and a memory 54. The tablet 20 has an application, also known as an app, saved in the memory 54 and executable by the processor 52 to enable the tablet to interface with the camera control unit as further illustrated below.

The tablet 20 is primarily operated by touching the screen, rather than using a physical keyboard, although a physical keyboard may be connectable to a tablet computer via a wired interface, such as a universal serial bus (USB) interface 56 or via a wireless interface 58, such as a Bluetooth interface. The tablet 20 is primarily operated by using an onscreen virtual keyboard, a passive stylus pen, or a digital pen.

The tablet 20 may have handwriting recognition. The tablets may also have one or more of the following: cameras, microphones, speakers, accelerometers, ambient light sensors, proximity sensors, global positioning systems. The tablet 20 has an operating system such as Windows, Linux, iOS, Android, or webOS. Examples of some tablets usable with the present invention include, without limitation, the Samsung Galaxy Tab, the ASUS Eee Pad Transformer, the HP TouchPad and the Apple iPad.

The present invention is directed to a system whereby the tablet 20 connects to and interfaces with the camera control unit 14. In an embodiment, the tablet 20 connects to and communicates with the camera control unit 14 and is powered and charged via a specialized isolated communication and power port. This enables a non-medical IEC60950 approved device to be used as a medical device, because the power is isolated to meet applicable medical device standards. In an embodiment, the tablet computer 20 connects and communicates with the camera control unit 14 via the USB interface 56. The tablet 20 may communicate with the camera control unit 14 using Ethernet over USB. The use of Ethernet over USB may require special drivers for the tablet 20 which may be included with the app.

Prior to operation, or upon the first connection between the tablet 20 and the camera control unit 14, the application is loaded onto the tablet. The application executes on the tablet to enable the tablet to securely interface with the camera control unit and to perform the functionality described below. In contrast to typical medical camera systems where the camera control unit does the processing and drives a monitor or touchscreen panel, the present invention is designed to take advantage of the memory and processing capabilities of a tablet in combination with the processing ability of the camera control unit.

Images may be transferred from the camera head 12 to the tablet 20. The images may be encrypted prior to transfer to the tablet 20 where the encrypted images are then decrypted. The tablet 20 allows for manipulation of images and video independently of the camera control unit, such as via scrolling and swiping the screen 50. The tablet 20 also allows for the images and/or video to be saved in different formats and/or to different places (e.g. internal memory, an external memory such as a USB memory device connectable to the tablet, or to a remote location over the Internet) independently of the camera control unit.

Preferably, the tablet 20 retains a database that may be synchronized with a database on the camera control unit 14. The database may store camera settings, image settings, video settings, and other information for a doctor, technician, nurse and patient. The database may also include settings for a specific facility and for a specific system. For example, the database may include settings for an entire system of connected devices, such as for example shaver pumps, electrosurgical tools, operating room electronics and the like.

The tablet 20, through a network interface 60, can access a local area network, a wide area network, or the Internet to obtain updates for the application on the tablet. Additionally, the tablet can obtain updates for the programs, firmware or settings of the camera control unit 14. Additionally, the tablet can obtain updates for the programs, firmware or settings of the camera head 12. In an embodiment, the updates for the camera head are passed to the camera control unit 14 for loading onto the camera head 12. In an alternative embodiment, the tablet 20 and the camera head 12 communicate directly, such as via a wireless network, and the tablet 20 updates the camera head 12 directly. The ability of the tablet 20 to obtain updates and operate independently of the camera control unit 14 allows for updates and evolution to the application(s) on the tablet without necessarily updating the software on either the camera control unit or the camera head.

In an additional embodiment, the system allows for synching by the camera control unit and/or the tablet to a remote database, such as a cloud-based database. The synchronization to the remote database may be done via a cellular connection, wired network connection such as Ethernet, or via a wireless connection such as Bluetooth, ZigBee or the like.

In an embodiment, the tablet has a front-facing camera 62 which allows for user images to be taken. Optionally, the tablet has a microphone 64 and/or speakers 66. In an embodiment, the tablet has face recognition programming and when first turned on, can recognize a user via the front facing camera 62 to automatically load settings and other information for that user. Face recognition may be used to authenticate users and may also be used to apply appropriate access and permissions to certain functionality of the system. User identification may also be accomplished, for example, using voice recognition programming via the microphone 64 or using fingerprint identification via the touchscreen 50 or via a fingerprint reader (not shown) integral to or attached to the tablet.

In an additional embodiment, the front-facing camera 62 allows for video-conferencing. Video-conferencing may utilize the monitor 18 either via picture-in-picture or via full screen. In an embodiment, the tablet 20 has a rear-facing camera 68 which may be used for example for allowing remote users with appropriate permission to view the operating room.

In an additional embodiment, the microphone 64 allows users to dictate notes which may be stored as audio files or that are converted into written notes via speech to text programming on the tablet. In an additional embodiment, the microphone 64 allows for users to recite commands to the tablet for control of the system. In an additional embodiment, the speakers 66 in the tablet allow for a user to hear comments, such as from others accessing the camera control unit remotely.

In an embodiment, the tablet 20 includes a global positioning system (GPS) 68. The GPS 68 may be used to disable the tablet if it is moved outside of a certain location. The GPS 68 may be used as part of the pairing and setup process between the tablet 20 and the camera control unit 14. Additionally, the GPS 68 may be used if the tablet is lost or misplaced to allow for erasure of the tablet, such as to comply with the privacy requirements of the Health Insurance Portability and Accountability act (HIPAA), and to help locate the tablet.

In an embodiment, video from the camera head 12, optionally with enhancements from the camera control unit 14, may be streamed to the tablet 20. The video from the camera head 12 may be encrypted, such as by the camera control unit 14 prior to being streamed to the tablet 20 where the encrypted video is then decrypted.

Preferably, when the tablet is linked to the camera control unit and configured, user preferences, such as those of the surgeon, are used to customize the images and video taken as well as the output to the monitor. Optionally, the camera control unit may modify the signal to the monitor to create a video overlay showing user preferences, settings, patient specific data and/or doctor specific data while a camera feed is displayed. Optionally, when video is being streamed to the tablet, the camera control unit may modify the signal to the monitor to indicate this.

In an additional embodiment of the present invention, the camera control unit 14 can be connected to other networked devices in the operating room and can serve as a network hub. The tablet 20 can then be used to control multiple devices and display information from multiple devices and may also pass information from multiple devices for simultaneous display on the monitor 18. Additionally, the tablet 20 may be used to control devices and display information from multiple devices directly using the wireless interface 58.

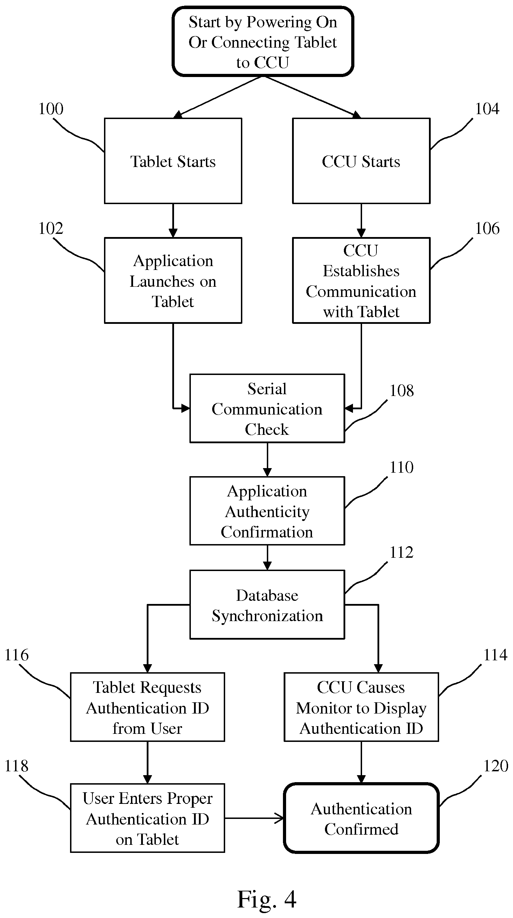

Operation of an embodiment of the present invention will now be described in relation to Fig. 4. When the camera control unit 14 is turned on (or when the tablet is connected to the camera control unit), power is supplied to the tablet 20 and causes the tablet 20 to turn on. Box 100. When the tablet 20 turns on, the application automatically loads, Box 102. In an embodiment, the loading of the application locks out all other applications and functions of the tablet 20; this prevents a user from inadvertently or intentionally launching another application. The camera control unit 14 also turns on, Box 104. The camera control unit 14 then establishes communication with the tablet 20, Box 106. The camera control unit 14 and the tablet 20 then perform a communication check, Box 108. The camera control unit 14 then verifies the authenticity of the application on the tablet, Box 110.

Once the authenticity of the application on the tablet is confirmed, databases on the camera control unit 14 and the tablet 20 are synchronized, Box 112. The camera control unit 14 then causes the monitor 18 to display an authentication identification for entry by the user on the tablet, Box 114. The tablet 20 prompts the user to enter the authentication identification, Box 116. Upon entry of the proper authentication identification by the user, Box 118, authentication is confirmed and the tablet 20 may be used with the camera control unit, Box 120.

Once the initial authentication is completed, future authentication is conducted using a certificate exchange. In the initial authentication, the tablet 20 requests a certificate from the camera control unit 14. The certificate is generated by the camera control unit 14 and sent to the tablet 20. In the future, the tablet 20 can provide the certificate to the camera control unit 14 for authentication.

In an embodiment of the present invention, the tablet 20 must initially be electrically connected to the camera control unit 14, such as via a USB cable. This prevents use of the tablet 20 from outside of an operating room, thereby minimizing the possibility of a change being made to the camera system settings in the midst of a procedure without the user making the changes being present, although this may also be prevented by using the tablet GPS 68. Additionally, requiring a physical connection between the tablet and the camera control units may be necessary in environments were wireless communication is not allowed. Additionally, requiring a physical connection between the tablet and the camera control unit ensures the tablet will have power.

In an embodiment, after the initial authentication is complete the tablet 20 may be physically disconnected from the camera control unit 14 and thereafter allowed to communicate wirelessly with the camera control unit, such as via Bluetooth, or 802.11 b/g/n. Allowing the tablet to be unplugged/undocked enables the operating room personnel to place the tablet it in a convenient location for use. In an additional embodiment, when the camera control unit 14 detects the presence of a tablet 20, the camera control unit queries the tablet to determine whether the app is compatible with the camera control unit and upgrades or downgrades the app on the tablet as necessary for compatibility.

During synchronization of the tablet 20 and camera control unit 14, the tablet and the camera control unit communicate data updates to each other based on which device has the latest updates. This minimizes the setup for service/replacement units, because a new unit connected to a previously updated tablet or camera control unit will be updated with the latest settings. The tablet may be used with multiple systems in an institution and all units may thereby have surgeon settings and other preferences synchronized.

Once authenticated, the tablet is used to control the system (in alternative embodiments, additional input devices can also be used to control the system independently of the tablet). Additionally, while one tablet is preferably used to control the system, additional tablets may connected to the camera control unit and may be used, such as for example, in a passive role for viewing and capturing images and/or video. For example, a tablet may be present at a nurse's station for viewing what is taking place in an operating room. In addition, a tablet, such as an iPad running a custom application, once authenticated, can receive image and video files. When such additional clients are attached to the camera control unit, notification can be displayed on the main surgical monitor and on the primary tablet.

In an embodiment, before the camera system 10 is used with a patient a case is selected. Optionally, without a case selection the camera system 10 is still functional and will produce a picture on the monitor. Optionally, the case information has already been input into the database accessible by the camera control unit 14 and the tablet 20 and may be selected by a user. Alternatively, new case information is entered into the database and the newly entered case is selected. Selection of a case allows for special settings and information for the patient to be loaded and used. Additionally, video and images may be captured during a procedure/surgery and linked to the case for later viewing. In an additional embodiment, the application on the tablet 20 creates a patient or surgeon report with specific attachments designated by the surgeon. The application may send an e-mail report to a patient and may generate a report file for uploading into another database or for storage.

Once a case is selected, a camera head 12 must be connected to the camera control unit 14 for the system 10 to be used. At the conclusion of a procedure the camera head 12 may be disconnected or the case terminated from the tablet 20. The tablet 20 can be used to view captured images and/or video, such as with a patient after a procedure/surgery.

Preferably a physical connection is required between the camera control unit 14 and the tablet 20 when the two are first paired together. Because the tablet is capable of wireless networking, it is important to ensure that the tablet is communicating with the proper camera control unit. Optionally, no physical connection is required and the camera control unit 14 searches for compatible tablets in its wireless vicinity; and upon finding a compatible tablet 20 in its wireless vicinity displays an authentication code on the monitor 18. The tablet 20 user would then enter the authentication code and authenticate the devices as discussed above.

Preferably, if the camera control unit 14 is physically connected to a tablet 20 with no special application thereon, nothing will happen. Alternatively, if the camera control unit 14 is physically connected to a tablet 20 with no special application thereon, the camera control unit will cause the tablet to display a message prompting a user to download the special application to the tablet. If the user elects to download the special application to the tablet, then the application may be downloaded from the camera control unit or from another accessible location.

Preferably, if a tablet with the proper application is physically connected to the camera control unit, then the application automatically launches. If a tablet has already been authenticated and is turned off, when the application is relaunched on the tablet, the application tries to connect with a particular camera control unit. If the tablet is unable to connect to the particular camera control unit, then the tablet displays a waiting screen. Upon subsequent communication with the particular camera control unit, the application automatically synchronizes and waits to be used with the system.

There is disclosed in the above description and the drawings, a medical camera system which fully and effectively overcomes the disadvantages associated with the prior art. However, it will be apparent that variations and modifications of the disclosed embodiments may be made without departing from the principles of the invention.

## Claims

1. A camera system (10) comprising:
a camera head (12);
a camera control unit (14) coupleable to the camera head (12);
a monitor (18) coupleable to the camera control unit (14) for displaying images from the camera head (12) and information from the camera control unit (14); and
a tablet computer (20) coupleable to the camera control unit (14) for control of the camera control unit (14) and the camera head (12), the tablet computer (20) further comprising an application that is authenticated by the camera control unit (14) for pairing the tablet computer (20) to the camera control unit (14)
**characterized in that** the tablet application locks out all other applications on the tablet computer (20) upon connection with the camera control unit (14).

2. The camera system (10) of claim 1 wherein the tablet computer (20) further comprises:
a memory for storing user information for use in controlling the camera control unit (14); a camera; and
face recognition software for automatically identifying a tablet computer (20) user and accessing the stored information for that user.

3. The camera system (10) of claim 2 wherein the memory for storing user information contains defined levels of authority and wherein once a user is identified using the face recognition software, the application loads the defined level of authority for that user and thereby limits the functionality of the application depending on the user.

4. The camera system (10) of claim 1 wherein the tablet computer (20) is initially coupled to the camera control unit (14) by a physical connection for authentication and thereafter the tablet computer (20) is coupleable to the camera control unit (14) wirelessly.

5. The camera system (10) of claim 1 wherein both the camera control unit (14) and the tablet computer (20) comprise a database having at least one of the group consisting of user, surgeon, facility and system settings and wherein the camera control unit (14) database and the tablet computer (20) database are synchronized when the camera control unit (14) and the tablet computer (20) are coupled together.

6. The camera system (10) of claim 5 further comprising a database remotely located from the camera control unit (14) and the tablet computer (20) and wherein the camera control unit database and the tablet computer (20) database are synchronized to the remotely located database.

7. The camera system (10) of claim 1 wherein a global positioning system is used to disable the tablet if the tablet is moved outside of a certain location.

8. The camera system (10) of claim 1 wherein the tablet computer (20) further comprises a camera and wherein:
the tablet computer (20) application allows for video conferencing using the tablet computer (20) camera and the monitor (18).

9. The camera system (10) of claim 1 wherein the tablet computer (20) receives encrypted images from the camera control unit (14) and the tablet computer (20) application decrypts the images and creates a patient or surgeon report with at least a subset of the images for display or communication.

10. The camera system (10) of claim 1 wherein the tablet computer (20) further comprises a global positioning system and pairing of the tablet computer (20) to the camera control unit (14) is dependent on the geographic location of the tablet computer (20).

11. The camera system (10) of claim 1 wherein the tablet computer (20) may view live images from the camera head (12); and wherein when the tablet computer (20) is displaying live images from the camera head (12) an indication is displayed on the monitor (18).

12. The camera system (10) of claim 1 wherein the tablet computer (20) transfers at least one of software and firmware updates to the camera control unit (14) and/or the camera head (12).

13. A method for authenticating a tablet computer (20) for use with a camera system (10) having a camera control unit (14), a monitor (18) and a tablet computer (20), the method comprising the steps of:
providing power to the camera control unit (14);
starting an application on the tablet computer (20);
establishing communication between the tablet computer (20) and the camera control unit (14);
determining the authenticity of the application on the tablet computer (20);
requesting authentication identification from the camera control unit (14);
displaying authentication identification on the monitor (18) by the camera control unit (14);
receiving the authentication identification on the tablet computer (20); and
issuing an authentication certificate to the tablet computer (20), the authentication certificate allowing for pairing of the camera control unit (14) and the tablet computer (20).

14. The method of claim 13 further comprising the steps of:
prior to establishing communication between the tablet computer (20) and the camera control unit (14), physically connecting the camera control unit (14) and the tablet computer (20) together; and
after issuing an authentication certificate to the tablet computer (20), physically disconnecting the camera control unit (14) and the tablet computer (20).

15. The method of claim 13 further comprising when the camera control unit (14) detects the presence of a tablet, the camera control unit (14) queries the tablet to determine whether the application is compatible with the camera control unit (14) and upgrading or downgrading the application as necessary for compatibility.

## Patentansprüche

1. Kamerasystem (10), das umfasst:
einen Kamerakopf (12);
eine Kamerasteuereinheit (14), die mit dem Kamerakopf (12) verbindbar ist;
einen Monitor (18), der mit der Kamerasteuereinheit (14) verbindbar ist, um Bilder vom Kamerakopf (12) und Informationen aus der Kamerasteuereinheit (14) anzuzeigen; und
einen Tablet-Computer (20), der mit der Kamerasteuereinheit (14) verbindbar ist, um die Kamerasteuereinheit (14) und den Kamerakopf (12) zu steuern, wobei der Tablet-Computer (20) ferner ein Anwendungsprogramm, das durch die Kamerasteuereinheit (14) authentifiziert wird, zum Paaren des Tablet-Computers (20) mit der Kamerasteuereinheit (14) umfasst,
**dadurch gekennzeichnet, dass** die Tablet-Anwendung alle anderen Anwendungen auf dem Tablet-Computer (20) bei Verbindung mit der Kamerasteuereinheit (14) sperrt.

2. Kamerasystem (10) nach Anspruch 1, wobei der Tablet-Computer (20) ferner umfasst:
einen Speicher zum Speichern von Benutzerinformationen zur Verwendung bei der Steuerung der Kamerasteuereinheit (14); eine Kamera; und
Gesichtserkennungssoftware zum automatischen Identifizieren eines Benutzers des Tablet-Computers (20) und Zugreifen auf die gespeicherten Informationen für diesen Benutzer.

3. Kamerasystem (10) nach Anspruch 2, wobei der Speicher zum Speichern von Benutzerinformationen definierte Berechtigungsebenen enthält und wobei das Anwendungsprogramm, nachdem ein Benutzer unter Verwendung der Gesichtserkennungssoftware identifiziert wurde, die definierte Berechtigungsebene für diesen Benutzer lädt und somit die Funktionalität des Anwendungsprogramms je nach Benutzer beschränkt.

4. Kamerasystem (10) nach Anspruch 1, wobei der Tablet-Computer (20) anfangs durch eine physische Verbindung zur Authentifizierung mit der Kamerasteuereinheit (14) verbunden wird und der Tablet-Computer (20) danach drahtlos mit der Kamerasteuereinheit (14) verbindbar ist.

5. Kamerasystem (10) nach Anspruch 1, wobei sowohl die Kamerasteuereinheit (14) als auch der Tablet-Computer (20) eine Datenbank umfasst, aufweisend zumindest eines aus der Gruppe aus Benutzer, Chirurgen, Standort und Systemeinstellungen , und wobei die Datenbank der Kamerasteuereinheit (14) und die Datenbank des Tablet-Computers (20) synchronisiert werden, wenn die Kamerasteuereinheit (14) und der Tablet-Computer (20) miteinander verbunden werden.

6. Kamerasystem (10) nach Anspruch 5, das ferner eine Datenbank umfasst, die von der Kamerasteuereinheit (14) und dem Tablet-Computer (20) entfernt gelegen ist, und wobei die Datenbank der Kamerasteuereinheit und die Datenbank des Tablet-Computers (20) mit der entfernt gelegenen Datenbank synchronisiert sind.

7. Kamerasystem (10) nach Anspruch 1, wobei ein globales Positionsbestimmungssystem verwendet wird, um das Tablet zu deaktivieren, wenn das Tablet außerhalb eines gewissen Orts bewegt wird.

8. Kamerasystem (10) nach Anspruch 1, wobei der Tablet-Computer (20) ferner eine Kamera umfasst und wobei:
das Anwendungsprogramm des Tablet-Computers (20) das Durchführen einer Videokonferenz unter Verwendung der Kamera des Tablet-Computers (20) und des Monitors (18) ermöglicht.

9. Kamerasystem (10) nach Anspruch 1, wobei der Tablet-Computer (20) verschlüsselte Bilder von der Kamerasteuereinheit (14) empfängt und das Anwendungsprogramm des Tablet-Computers (20) die Bilder entschlüsselt und einen Patienten- oder Chirurgenbericht mit zumindest einer Teilmenge der Bilder zur Anzeige oder Kommunikation erstellt.

10. Kamerasystem (10) nach Anspruch 1, wobei der Tablet-Computer (20) ferner ein globales Positionsbestimmungssystem umfasst und das Paaren des Tablet-Computers (20) mit der Kamerasteuereinheit (14) vom geographischen Ort des Tablet-Computers (20) abhängt.

11. Kamerasystem (10) nach Anspruch 1, wobei der Tablet-Computer (20) Live-Bilder vom Kamerakopf (12) darstellen kann; und wobei, wenn der Tablet-Computer (20) Live-Bilder vom Kamerakopf (12) anzeigt, ein Hinweis auf dem Monitor (18) angezeigt wird.

12. Kamerasystem (10) nach Anspruch 1, wobei der Tablet-Computer (20) zumindest eines von Software- und Firmwareaktualisierungen an die Kamerasteuereinheit (14) und/oder den Kamerakopf (12) überträgt.

13. Verfahren zum Authentifizieren eines Tablet-Computers (20) zur Verwendung mit einem Kamerasystem (10), das eine Kamerasteuereinheit (14), einen Monitor (18) und einen Tablet-Computer (20) aufweist, wobei das Verfahren die Schritte umfasst:
Versorgen der Kamerasteuereinheit (14) mit Energie;
Starten eines Anwendungsprogramms auf dem Tablet-Computer (20) ;
Aufbauen einer Kommunikation zwischen dem Tablet-Computer (20) und der Kamerasteuereinheit (14);
Ermitteln der Authentizität des Anwendungsprogramms auf dem Tablet-Computer (20);
Anfordern einer Authentifizierungskennung von der Kamerasteuereinheit (14);
Anzeigen einer Authentifizierungskennung auf dem Monitor (18) durch die Kamerasteuereinheit (14);
Empfangen der Authentifizierungskennung auf dem Tablet-Computer (20); und
Ausgeben eines Authentifizierungszertifikats an den Tablet-Computer (20), wobei das Authentifizierungszertifikat das Paaren der Kamerasteuereinheit (14) und des Tablet-Computers (20) ermöglicht.

14. Verfahren nach Anspruch 13, das ferner die Schritte umfasst:
vor dem Aufbauen einer Kommunikation zwischen dem Tablet-Computer (20) und der Kamerasteuereinheit (14) physisches Verbinden der Kamerasteuereinheit (14) und des Tablet-Computers (20) miteinander; und
nach Ausgeben eines Authentifizierungszertifikats an den Tablet-Computer (20) physisches Trennen der Kamerasteuereinheit (14) und des Tablet-Computers (20).

15. Verfahren nach Anspruch 13, das ferner umfasst, dass, wenn die Kamerasteuereinheit (14) das Vorhandensein eines Tablets erkennt, die Kamerasteuereinheit (14) das Tablet abfragt, um zu ermitteln, ob das Anwendungsprogramm mit der Kamerasteuereinheit (14) kompatibel ist, und das Upgraden oder Downgraden des Anwendungsprogramms wie für eine Kompatibilität erforderlich umfasst.

## Revendications

1. Système de caméra (10) comprenant :
une tête de caméra (12) ;
une unité de commande de caméra (14) pouvant être couplée à la tête de caméra (12) ;
un moniteur (18) pouvant être couplé à l'unité de commande de caméra (14) pour l'affichage d'images de la tête de caméra (12) et d'informations de l'unité de commande de caméra (14) ; et
une tablette (20) pouvant être couplée à l'unité de commande de caméra (14) pour la commande de l'unité de commande de caméra (14) et de la tête de caméra (12), la tablette (20) comprenant, en outre, une application qui est authentifiée par l'unité de commande de caméra (14) pour le jumelage de la tablette (20) à l'unité de commande de caméra (14)
**caractérisé en ce que** l'application de tablette bloque toutes les autres applications sur la tablette (20) lors de la connexion avec l'unité de commande de caméra (14).

2. Système de caméra (10) selon la revendication 1, dans lequel la tablette (20) comprend en outre :
une mémoire pour le stockage d'informations d'utilisateurs pour une utilisation dans la commande de l'unité de commande de caméra (14) ; une caméra ; et
un logiciel de reconnaissance de visages pour l'identification automatique d'un utilisateur de la tablette (20) et l'accès aux informations stockées de cet utilisateur.

3. Système de caméra (10) selon la revendication 2, dans lequel la mémoire pour le stockage d'informations d'utilisateurs contient des niveaux d'autorité définis et dans lequel une fois qu'un utilisateur est identifié en utilisant le logiciel de reconnaissance de visages, l'application charge le niveau d'autorité défini pour cet utilisateur et limite ainsi la fonctionnalité de l'application en fonction de l'utilisateur.

4. Système de caméra (10) selon la revendication 1, dans lequel la tablette (20) est initialement couplée à l'unité de commande de caméra (14) par une connexion physique pour l'authentification et ensuite la tablette (20) peut être couplée sans fil à l'unité de commande de caméra (14).

5. Système de caméra (10) selon la revendication 1, dans lequel tant l'unité de commande de caméra (14) que la tablette (20) comprennent une base de données ayant au moins un du groupe constitué d'utilisateur, de chirurgien, d'installation et de paramètres de système et dans lequel la base de données de l'unité de commande de caméra (14) et la base de données de la tablette (20) sont synchronisées lorsque l'unité de commande de caméra (14) et la tablette (20) sont couplées ensemble.

6. Système de caméra (10) selon la revendication 5, comprenant, en outre, une base de données située à distance de l'unité de commande de caméra (14) et de la tablette (20) et dans lequel la base de données de l'unité de commande de caméra et la base de données de la tablette (20) sont synchronisées avec la base de données située à distance.

7. Système de caméra (10) selon la revendication 1, dans lequel un système de positionnement mondial est utilisé pour désactiver la tablette si la tablette est déplacée en dehors d'une certaine zone.

8. Système de caméra (10) selon la revendication 1, dans lequel la tablette (20) comprend en outre une caméra et dans lequel :
l'application de la tablette (20) permet les vidéoconférences utilisant la caméra de la tablette (20) et le moniteur (18).

9. Système de caméra (10) selon la revendication 1, dans lequel la tablette (20) reçoit des images chiffrées de l'unité de commande de caméra (14) et l'application de la tablette (20) déchiffre les images et crée un rapport de patient ou de chirurgien avec au moins un sous-ensemble des images pour l'affichage ou la communication.

10. Système de caméra (10) selon la revendication 1, dans lequel la tablette (20) comprend en outre un système de positionnement mondial et le jumelage de la tablette (20) à l'unité de commande de caméra (14) dépend de la localisation géographique de la tablette (20).

11. Système de caméra (10) selon la revendication 1, dans lequel la tablette (20) peut visualiser des images en direct à partir de la tête de caméra (12) ; et dans lequel, lorsque la tablette (20) affiche des images en direct à partir de la tête de caméra (12), une indication est affichée sur le moniteur (18).

12. Système de caméra (10) selon la revendication 1, dans lequel la tablette (20) transfert au moins une de mises à jour de logiciel et de firmware à l'unité de commande de caméra (14) et/ou à la tête de caméra (12).

13. Procédé pour l'authentification d'une tablette (20) pour une utilisation avec un système de caméra (10) ayant une unité de commande de caméra (14), un moniteur (18) et une tablette (20), le procédé comprenant les étapes consistant de :
l'alimentation en électricité de l'unité de commande de caméra (14) ;
le démarrage d'une application sur la tablette (20) ;
l'établissement de la communication entre la tablette (20) et l'unité de commande de caméra (14) ;
la détermination de l'authenticité de l'application sur la tablette (20) ;
la demande d'identification d'authenticité à partir de l'unité de commande de caméra (14) ;
l'affichage de l'identification d'authenticité sur le moniteur (18) par l'unité de commande de caméra (14) ;
la réception de l'identification d'authenticité sur la tablette (20) ; et
l'émission d'un certification d'authenticité à la tablette (20), le certificat d'authenticité permettant le jumelage de l'unité de commande de caméra (14) et de la tablette (20).

14. Procédé selon la revendication 13, comprenant en outre, les étapes consistant de :
avant l'établissement de la communication entre la tablette (20) et l'unité de commande de caméra (14), la connexion physique ensemble de l'unité de commande de caméra (14) et de la tablette (20) ; et
après l'émission d'un certification d'authenticité à la tablette (20), la déconnexion physique de l'unité de commande de caméra (14) et de la tablette (20).

15. Procédé selon la revendication 13, comprenant en outre, lorsque l'unité de commande de caméra (14) détecte la présence d'une tablette, l'unité de commande de caméra (14) interroge la tablette pour déterminer si l'application est compatible avec l'unité de commande de caméra (14) et la mise à niveau ou le déclassement de l'application selon les besoins pour la compatibilité.
